# EUROPEAN PATENT SPECIFICATION

(11) **EP 1 004 676 B1**
(45) Date of publication and mention of the grant of the patent: **15.03.2006**
(21) Application number: 98936732.1
(22) Date of filing: 10.08.1998
(51) Int. Cl.: C12Q 1/68

(54) **METHODS FOR DNA AMPLIFICATION AND KITS THEREFOR**
METHODEN ZUR DNS VERVIELFÄLTIGUNG UND MATERIAL DAFÜR
METHODES D'AMPLIFICATION D'ADN ET MATERIELS AFFERENTS

(30) Priority: 14.08.1997 JP 23188597; 21.10.1997 JP 30501697
(43) Date of publication of application: 31.05.2000
(73) Proprietor: TAKARA BIO INC., Otsu-shi, Shiga-ken (JP)
(72) Inventor: YAMAMOTO, Junko, Shiga 520-0831 (JP); MUKAI, Hiroyuki, Moriyama-shi Shiga 524-0102 (JP); HINO, Fumitsugu, Shiga 525-0028 (JP); KATO, Ikunoshin, Kyoto 611-0028 (JP)
(74) Representative: Schüssler, Andrea
(86) International application number: PCT/JP1998/003566
(87) International publication number: WO 1999/009213

(56) References cited:
- EP-A- 0 610 615
- JP-A- 5 261 000
- JP-A- 10 066 596
- JP-T- 7 506 245
- JP-T- 7 509 368
- US-A- 5 618 703
- CHESTER N ET AL: "DIMETHYL SULFOXIDE-MEDIATED PRIMER TM REDUCTION: A METHOD FOR ANALYZING THE ROLE OF RENATURATION TEMPERATURE IN THE POLYMERASE CHAIN REACTION" ANALYTICAL BIOCHEMISTRY,US,ACADEMIC PRESS, SAN DIEGO, CA, vol. 209, no. 2, 1 March 1993 (1993-03-01), pages 284-290, XP000349766 ISSN: 0003-2697
- SIDHU MANINDER K ET AL: "Dimethyl sulfoxide improves RNA amplification." BIOTECHNIQUES, vol. 21, no. 1, 1996, pages 44-47, XP000999068 ISSN: 0736-6205
- SEELA F ET AL: "7-DEAZAPURINE CONTAINING DNA: EFFICIENCY OF C7GDTP, C7ADTP AND C7IDTP INCORPORATION DURING PCR-AMPLIFICATION AND PROTECTION FROM ENDODEOXYRIBONUCLEASE HYDROLYSIS" NUCLEIC ACIDS RESEARCH,GB,OXFORD UNIVERSITY PRESS, SURREY, vol. 20, no. 1, 1992, pages 55-61, XP002044088 ISSN: 0305-1048
- INNIS M A: "PCR WITH 7 DEAZA-2'-DEOXYGUANOSINE TRIPHOSPHATE" INNIS, M. A., ET AL. (ED.). PCR PROTOCOLS: A GUIDE TO METHODS AND, 1990, pages 54-59, XP000999096 USA;LONDON, ENGLAND, UK. ILLUS. ISBN 0-12-372181-4(PAPER); ISBN 0-12-372180-6(CLOTH). 1990
- NUCLEIC ACIDS RESEARCH, Vol. 24, No. 24, (1996), AUER T. et al., "Selective Amplification of RNA Utilizing the Nucleotide Analog dITP and Thermus Thermophilus DNA Polymerase", p. 5021-5025, XP002916438

## Description

### TECHNICAL FIELD

The present invention relates to a method for amplifying DNA, particularly to RT-PCR method capable of selectively amplifying only a nucleotide sequence corresponding to RNA even in admixture of DNAs, and to a kit usable for the method for amplifying DNA.

### BACKGROUND ART

A method for amplifying DNA, particularly polymerase chain reaction (PCR) method, is a technique for simply amplifying a desired nucleic acid fragment in vitro, and presently has been an indispensable experimental method in a wide variety of fields of biology, medicine, agriculture, and the like, as well as in the studies relating to genes. PCR method is originally a technique for amplifying DNA, and there has been developed PCR method for amplifying a DNA fragment having a nucleotide sequence corresponding to RNA, which is so-called RT-PCR method. The method is a method for specifically amplifying cDNA derived from RNA, comprising synthesizing a DNA transcriptional product which is complementary to RNA (cDNA) by using a reverse transcriptase having a RNA-dependent DNA polymerase activity, namely a reverse transcription activity, wherein the reverse transcriptase is a kind of a DNA polymerase, or using a DNA polymerase also having a reverse transcription activity, and subsequently carrying out PCR by the use of the resulting transcription product as a template. Aside from the fact that RT-PCR method is utilizable for cloning of cDNA derived from mRNA and for preparation of a cDNA library, it is also useful as a method for examining an expression state of a particular mRNA.

However, when DNA is admixed in an RNA sample to be used in RT-PCR, since products in which a certain region on DNA encoding RNA and a pseudogene are used as templates for PCR are simultaneously amplified, it would be difficult to selectively obtain only the amplified product by the use of RNA as a template. In order to prevent the formation of an amplified product derived from DNA admixed in such an RNA sample, it would be necessary to use a purified RNA as a sample, or to use a sample in which an admixed DNA is removed by DNA degradation enzyme treatment, or the like, thereby making its procedures complicated.

As a method for solving this problem, there has been proposed a method for selectively amplifying a DNA fragment derived from cDNA which is complementary to RNA, comprising synthesizing an RNA-cDNA double-stranded nucleic acid of which *Tm* value, i.e. melting temperature, is low, by the reverse transcription reaction in the presence of a nucleotide analog dITP, and further carrying out PCR reaction at a low-temperature of denaturation temperature in the presence of the same nucleotide analog, thereby suppressing the amplification of a product caused by DNA admixed in a sample *[Nucl. Acids Res.* 24, 5021-5025 (1996) and US 5, 618, 703]. However, this method needs to change the amount of dITP and the reaction temperature depending upon a GC content of an amplified nucleotide sequence, thereby making it difficult to find the optimal conditions. Further, it is not satisfactory in its reactivities, and in some cases selective amplification does not take place depending on a target nucleotide sequence.

### DISCLOSURE OF INVENTION

In view of overcoming the defects in the prior art, an object of the present invention is to provide a method for amplifying DNA capable of selectively obtaining only a DNA fragment having the nucleotide sequence corresponding to RNA even in admixture of DNA, the method having excellent wide utility, reproducibility and detection sensitivity.

The present inventors have elucidated that one of the problems of the method using the above nucleotide analog resides in that it has been difficult to set temperature conditions such that an RNA-cDNA double-stranded nucleic acid comprising a template RNA and cDNA to which a nucleotide analog is incorporated is denatured, and that a usual double-stranded DNA is not denatured. Moreover, they have found that this problem can be solved by adding a compound for lowering *Tm* value of an RNA-cDNA double-stranded nucleic acid during DNA amplification reaction, the compound including, for instance, formamide, and the like.

Further, the present inventors have considered that another problem of the above method is that the incorporation of a nucleotide analog into the DNA strand synthesized in the above method is affected by a nucleotide sequence of a target RNA, particularly by its GC content. The present inventors have found that DNA amplification takes place with excellent reproducibility regardless of the GC content of a target RNA by adding to the reaction mixture two or more kinds of nucleotide analogs to be incorporated into DNA at a given frequency, and the present invention has been completed thereby.

In sum, the present invention is concerned with:
[1] a method for amplifying a DNA by the use of a DNA fragment comprising a nucleotide analog as a template in the presence of nucleotide analogs, characterized in that the method for amplifying a DNA is carried out in the presence of 7-deaza-dGTP and 7-Deaza dATP or dITP and 7-Deaza-dATP; and, optionally, in the presence of a compound for lowering *Tm* value of a double-stranded nucleic acid;
[2] a method for amplifying a DNA by the use of a DNA fragment comprising a nucleotide analog as a template in the presence of nucleotide analogs, characterized in that the method for amplifying a DNA is carried out in the presence of one or more kinds of nucleotide analogs and a compound for lowering *Tm* value of a double-stranded nucleic acid;
[3] a kit for amplifying a DNA in the presence of a nucleotide analog by the use of a DNA fragment comprising a nucleotide analog as a template, characterized in that the kit comprises 7-deaza-dGTP and 7-Deaza dATP or dITP and 7-Deaza-dATP; and, optionally, a compound for lowering *Tm* value of a double-stranded nucleic acid; and
[4] a kit for amplifying a DNA in the presence of a nucleotide analog by the use of a DNA fragment comprising a nucleotide analog as a template, characterized in that the kit comprises one or more kinds of nucleotide analogs and a compound for lowering *Tm* value of a double-stranded nucleic acid.

### BEST MODE FOR CARRYING OUT THE INVENTION

One of the great features of the method for amplifying a DNA of the present invention resides in that, in the method for amplifying a DNA by the use of a DNA fragment comprising a nucleotide analog as a template, the method being carried out in the presence of nucleotide analogs, the method for amplifying DNA is carried out in the presence of two or more kinds of nucleotide analogs and/or in the presence of a compound for lowering *Tm* value of a double-stranded nucleic acid.

In the method of the present invention, as a DNA fragment to be used as a template for amplification reaction, there can be used cDNA prepared by reverse transcription reaction by the use of RNA as a template in the presence of one or more kinds of nucleotide analogs. The reverse transcription reaction may be carried out in further presence of a compound for lowering *Tm* value of a double-stranded nucleic acid.

A sample comprising RNA to which the method of the present invention can be applied is not particularly limited. Examples thereof include biologically derived samples such as cells, tissues and blood, and samples having possibility of containing organisms, the samples including foods, soils and wastewater. In addition, a nucleic acid-containing preparation obtainable by treating the above samples by a known method may be also used. As the preparations, there can be used in the present invention, for instance, samples obtainable from cell disrupted products or fractionated products thereof, samples rich in whole RNAs, or particular RNA molecules, for instance, mRNA in the samples, and the like. Incidentally, DNA corresponding to RNA can be selectively amplified in these samples without being affected from coexisting DNAs, even if DNAs were contained in these samples. In addition, there may be used samples from which DNA is removed by a known method.

The RNA to be amplified by the method of the present invention is not particularly limited. Examples thereof include an RNA molecule such as whole RNA, mRNA, tRNA and rRNA, or a particular RNA molecule, including a given RNA that can be used to prepare a primer used in reverse transcription and amplification reactions. For instance, as long as the presence or absence of the target RNA and the increase or decrease in its level indicate a particular disease, the diagnosis of the disease can be made by the method of the present invention. In addition, the presence of a microorganism in a sample can be detected by using the microorganism-specific RNA as a target. The used amount of RNA to be amplified in one reverse transcription reaction may be preferably an amount sufficient for carrying out reverse transcription reaction. For instance, from the viewpoint of the sensitivity, the amount is preferably 0.1 µg or more, more preferably 0.5 µg or more, and from the viewpoint of the reaction inhibition, the amount is preferably 2 µg or less, more preferably 1 µg or less.

The primer used in cDNA synthesis from a target RNA in the present invention is an oligonucleotide having a nucleotide sequence complementary to the target RNA, and the primer is not particularly limited as long as it anneals against the target RNA in operable reaction conditions. The primer may be oligonucleotides such as oligo(dT) and oligonucleotide(random primer) having a random sequence.

The length of the primer is preferably 6 nucleotides or more, more preferably 10 nucleotides or more, from the viewpoint of carrying out specific annealing, and the length is preferably 100 nucleotides or less, more preferably 30 nucleotides or less, from the viewpoint of the oligonucleotide synthesis. The oligonucleotide can be synthesized by, for instance, phosphoamidite method using DNA Synthesizer Model 394 manufactured by ABI (Applied Biosystems Inc.). Besides the above, oligonucleotides synthesized by any of methods such as triphosphate method, H-phosphonate method and thiophosphite method may be used. In addition, it may be biological sample-derived oligonucleotides, including, for instance, those prepared by isolating from a restriction endonuclease digest of DNAs prepared from the sample. The used amount of the primer in a reverse transcription reaction mixture may be an amount sufficient for carrying out cDNA synthesis from a target RNA. From the viewpoint of the synthesis efficiency, the amount is preferably 0.1 µM or more, more preferably 0.5 µM or more, and from the viewpoint of the reaction inhibition, the amount is preferably 10 µM or less, more preferably 5 µM or less.

In addition, the primer used in amplification reaction of DNA (PCR) is also not particularly limited, as long as the primer can be used in amplification of DNA by the use of cDNA corresponding to a target RNA as a template, and various oligonucleotides similar to ones mentioned above can be used. The primer used in the cDNA synthesis from the above target RNA may be used in the amplification reaction of DNA. The used amount of the above primer in a PCR mixture may be an amount sufficient for carrying out synthesis for double-stranded DNA by the use of a template cDNA obtained from a target RNA as a template. From the viewpoint of the synthesis efficiency, the amount is preferably 0.05 µM or more, more preferably 0.1 µM or more, and from the viewpoint of the reaction specificity, the amount is preferably 2 µM or less, more preferably 1 µM or less.

The enzyme having a reverse transcription activity usable in the present invention is not particularly limited, as long as the enzyme has a cDNA synthesizing activity by the use of RNA as a template. For instance, there can be included reverse transcriptases of various origins, including avian myeloblastosis virus-derived reverse transcriptase (AMV-RTase), Moloney's mouse leukemia virus-derived reverse transcriptase (MMLV-RTase), Raus-associated virus 2-derived reverse transcriptase (RAV-2-RTase), and the like. Besides the above, DNA polymerases also having a reverse transcription activity can be used. For instance, there can be used *Thermus thermophilus*-derived DNA polymerase (Tth DNA polymerase), *Bacillus caldotenax*-derived DNA polymerase (Bca DNA polymerase), and the like. AMV-RTase or RAV-2-RTase is preferable. These enzymes may be any of those obtained by purifying from the natural origins, or recombinant proteins produced by genetic engineering means. The used amount of the enzyme having a reverse transcription activity may be an amount sufficient for carrying out reverse transcription reaction.

One of the great features of the method for amplifying a DNA of the present invention resides in that DNA amplification reaction is carried out by the use of cDNA formed by the reverse transcription reaction as a template, thereby amplifying the DNA. As DNA amplification reaction, PCR method (Japanese Examined Patent Publication Nos. Hei 4-67957 and 4-67960) is preferably used.

As DNA polymerases usable in PCR method, various thermostable DNA polymerases can be used, including, for instance, *Thermus thermophilus*-derived DNA polymerase (Tth DNA polymerase), *Thermus aquatious*-derived DNA polymerase (Taq DNA polymerase), *Pyrococcus furiosus*-derived DNA polymerase (Pfu DNA polymerase), and other thermostable DNA polymerases. These enzymes may be used alone or in admixture. These enzymes may be any of proteins obtained by purifying from the natural origins, or recombinant proteins produced by genetic engineering means.

In addition, when a DNA polymerase also having the reverse transcription activity mentioned above is used as a reverse transcriptase, since the same DNA polymerase can be reused also in PCR reaction, the reverse transcription reaction and the PCR reaction can be carried out by using a single reaction tube.

The amount of the DNA polymerase may be an amount usually employed when polymerase reaction is carried out.

The nucleotide analog described in the present specification refers to a substance other than dATP, dCTP, dGTP or dTTP, wherein the substance is incorporated into cDNA freshly synthesized in cDNA synthesis reaction by the use of RNA as a template and in DNA amplification reaction by the use of the cDNA as a template, and does not cause to stop the reaction. Particularly, there can be used in the present invention nucleotide analogs having a property of lowering *Tm* value of DNA by incorporation of these nucleotide analogs. The nucleotide analog used in the present invention is not particularly limited, and there can be used 7-Deaza-dGTP, 7-Deaza-dATP, dITP, hydroxymethyl dUTP, and the like.

In cDNA synthesis and DNA amplification using one kind of a nucleotide analog, the incorporation frequency of a nucleotide analog may be affected depending upon the GC content of RNA to be targeted in some cases. For instance, dITP is incorporated in a DNA strand in place of dGTP, and there may be some cases where the higher the GC content of a target RNA, the more its incorporation frequency, even under the same reaction conditions. In the present invention, the conditions for selective amplification of a product corresponding to an RNA can be easily set by using two or more kinds of appropriate nucleotide analogs in combination, so that the combined incorporation frequency of these nucleotide analogs is not be influenced by the GC content of the target RNA.

The combination of nucleotide analogs as described above is not particularly limited, and two or more kinds of nucleotide analogs which are likely to be incorporated uniformly can be used in appropriate combination. For instance, there can be used in the present invention a combination of a nucleotide analog to be incorporated in a DNA strand in place of dATP or dTTP with a nucleotide analog to be incorporated in a DNA strand in place of dCTP or dGTP. In particular, a combination of 7-Deaza-dATP and 7-Deaza-dGTP can be preferably used.

The content of a nucleotide analog in a reverse transcription reaction mixture is preferably 50 µM or more, more preferably 55 µM or more, from the viewpoint of sufficiently exhibiting an effect of lowering *Tm* value, and the content is preferably 1.5 mM or less, more preferably 200 µM or less, from the viewpoint of the reaction efficiency. In this case, it is desired that the content of dNTP is 200 µM or more and 1 mM or less, respectively.

The content of a nucleotide analog in a reaction mixture for PCR is preferably 10 µM or more, more preferably 11 µM or more, from the viewpoint of sufficiently exhibiting an effect of lowering *Tm* value, and the content is preferably 1.5 mM or less, more preferably 200 µM or less, from the viewpoint of the reaction efficiency. In this case, it is desired that the content of dNTP is 200 µM or more and 1 mM or less, respectively.

In the present specification, the term "a compound for lowering *Tm* value of a double-stranded nucleic acid" refers to a substance other than that incorporated in a nucleic acid as.a.substrate during cDNA synthesis and DNA amplification reaction, which acts to lower a dissociation temperature (Tm value) for denaturing a double-stranded nucleic acid into a single strand in the presence of the substance, the double-stranded nucleic acid including, for instance, a double-stranded DNA, an RNA-DNA double-stranded nucleic acid constituted by RNA and DNA, or the like. As such compounds, there can be used organic solvents such as formamide and dimethyl sulfoxide (DMSO) and amphoteric electrolytes (betains) represented by trimethyl glycine. In particular, formamide is preferably used in the present invention because it is said not to give mal-affects to the extension activity of a DNA polymerase. In addition, these compounds can be used in admixture of two or more kinds. For instance, favorable results can be obtained by using formamide and dimethyl sulfoxide in combination.

In addition, even if the nucleotide analog were a single kind, there are some cases where there is no influence by the GC content depending on the kind of RNA used as a template. In such a case, more favorable results can be obtained by further using a compound for lowering *Tm* value of a double-stranded nucleic acid, and such an embodiment is also within the scope of the present invention.

The content of a compound for lowering *Tm* value of a double-stranded nucleic acid in a reverse transcription reaction mixture is preferably 20% by weight or less, more preferably 10% by weight or less, from the viewpoint of the reaction inhibition. In addition, during PCR reaction, the content of the compound in a reaction mixture for PCR is preferably 0.5% by weight or more, more preferably 1% by weight or more, from the viewpoint of lowering *Tm* value, and the content is preferably 20% by weight or less, more preferably 15% by weight or less, from the viewpoint of the reaction inhibition.

Concretely, when formamide is used, the content of formamide in a reverse transcription reaction mixture is preferably 20% by weight or less, more preferably 10% by weight or less, from the viewpoint of the reaction inhibition. In addition, the content of formamide in a reaction mixture for PCR is preferably 1% by weight or more, more preferably 1.5% by weight or more, from the viewpoint of lowering *Tm* value, and the content is preferably 20% by weight or less, more preferably 15% by weight or less, from the viewpoint of the reaction inhibition.

Reverse transcription from RNA to cDNA is carried out in a reaction mixture comprising a sample containing a target RNA, a primer which is an oligonucleotide having a sequence complementary to the target, four kinds of deoxynucleotide triphosphates, an enzyme having a reverse transcription activity and at least one kind of a nucleotide analog. By this reaction, DNA having a nucleotide sequence complementary to the target RNA sequence mentioned above (cDNA) comprising a nucleotide analog can be synthesized. In addition, the resulting template RNA-cDNA double-stranded nucleic acid to be formed from cDNA and the target RNA has lower *Tm* value, as compared to that of a double-stranded nucleic acid without containing a nucleotide analog. For instance, there is exhibited an excellent property of dissociating into a single strand at a lower temperature, as compared to a usual double-stranded DNA without containing a nucleotide analog.

Next, a DNA fragment derived from cDNA can be amplified by carrying out the DNA amplification reaction, including, for instance, PCR, by the use of cDNA mentioned above as a template. In this case, there can be selectively amplified a DNA fragment corresponding to cDNA, i.e. a DNA fragment corresponding to a target RNA, by carrying out DNA amplification reaction in the presence of a nucleotide analog under conditions that the template RNA-cDNA double-stranded nucleic acid mentioned above is denatured, and that a double-stranded DNA without containing a nucleotide analog admixed in a reaction mixture is not denatured, the conditions including, for instance, in PCR, setting a temperature for denaturation process. However, when various RNAs having different nucleotide sequences and chain lengths are used as targets, since the incorporation frequency of a nucleotide analog during the reverse transcription reaction varies depending on each target RNA, it may be difficult in some cases to set generalized conditions. In such a case, the conditions in which the selective amplification mentioned above takes place can be easily set by further adding to a DNA amplification reaction mixture a compound for lowering *Tm* value of a double-stranded nucleic acid as described above, including, for instance, formamide. For instance, when PCR is carried out by adding formamide to have a final concentration of 1% by weight to 20% by weight, only the amplified product corresponding to RNA can be obtained by setting a denaturation temperature of 80° to 85°C. Concretely, in the embodiment of the method for amplifying a DNA of the present invention, the method is carried out in a DNA amplification reaction mixture in the presence of two or more kinds of nucleotide analogs or in the presence of a compound for lowering *Tm* value of a double-stranded nucleic acid. Particularly, an embodiment in which a nucleotide analog and a compound for lowering *Tm* value are coexistent is preferable. In this case, a nucleotide analog existing therein may be one or more kinds. A nucleotide analog is added to have the concentration mentioned above when a reaction mixture for PCR is prepared. When a reaction mixture for PCR is prepared by taking a part of the solution obtained after reverse transcription reaction, there may be used as a reaction mixture for PCR by adding only the components other than a nucleotide analog to the mixture, as long as a necessary amount of the nucleotide analogs is contained in the reverse transcription reaction mixture.

In the method of the present invention, cDNA synthesis reaction by the use of RNA as a template and DNA amplification reaction by the use of the cDNA as a template can be consecutively carried out using the same reaction mixture. In this case, a reaction mixture comprising both an enzyme having a reverse transcription activity and a DNA polymerase usable in PCR is used. Such a reaction mixture can be prepared by a known method and used [*Bio Techniques* **18**, 678-687 (1995)]. Further, the same enzyme as that used in reverse transcription reaction can be also used for cDNA synthesis reaction and DNA amplification reaction by using a thermostable DNA polymerase having a reverse transcription activity.

The kit of the present invention is a kit usable for the above method, wherein the kit is capable of selectively amplifying DNA having a nucleotide sequence corresponding to RNA. Such a kit includes ones comprising one or more kinds of nucleotide analogs and a compound for lowering *Tm* value of a double-stranded nucleic acid, or ones comprising 7-Deazu-dGTP and 7-deazu-dATP or dITP and 7-Deazu-dATP; and, optionally, a compound for lowering *Tm* value of a double-stranded nucleic acid. Among them, the kit comprising two or more kinds of nucleotide analogs and a compound for lowering *Tm* value of a double-stranded nucleic acid is particularly preferable. The kit may comprise an enzyme having reverse transcriptase activity, a reaction buffer used in the enzyme reaction, a DNA polymerase usable in the DNA amplification reaction, and other reagents. In addition, the nucleotide analogs and the compound for lowering *Tm* value of a double-stranded nucleic acid mentioned above may be in a state of being added to a reaction buffer, or the like, to have an appropriate concentration upon use. By using such a kit, it is made possible to easily carry out selective amplification of only the DNA fragment having a nucleotide sequence corresponding to RNA from the RNA sample admixed with DNA.

### EXAMPLES

Next, the present invention will be described more concretely by means of the working examples, without intending to restrict the scope of the present invention to these working examples.

### Example 1: Preparation of RNA and DNA

Each of RNAs and DNAs used in the following experiment was prepared respectively from human cultured cells HL-60 (ATCC CRL-1964) or human cultured cells HT-29 (ATCC HTB-38), respectively, using a TRIzol Reagent (manufactured by LIFE TECHNOLOGIES) in accordance with the procedures described in manual attached to the reagent. RNA derived from human cultured cells HL-60 had purity OD₂₆₀/OD₂₈₀ of 1.8 or more, and DNA had purity OD₂₆₀/OD₂₈₀ of 1.7 or more. In addition, RNA derived from human cultured cells HT-29 had purity OD₂₆₀/OD₂₈₀ of 1.8 or more, and DNA had purity OD₂₆₀/OD₂₈₀ of 1.7 or more.

### Example 2: RT-PCR for various RNAs as Target

Selective amplification was tried with mRNAs having different chain lengths and GC contents as targets. There were selected as targets a region consisting of 621 bases (GC content: 38%) in transferrin receptor mRNA, a region consisting of 252 bases (GC content: 45%) in cytokeratin mRNA, and a region consisting of 275 bases (GC content: 58%) in β-actin mRNA. In the amplification of the transferrin receptor mRNA and the β-actin mRNA, RNA. derived from human cultured cells HL-60 was used as a template, and in the amplification of the cytokeratin mRNA, RNA derived from human cultured cells HT-29 was used as a template, respectively.

As the primers for amplifying the above targets, primer P5 and primer P6 (SEQ ID NOs: 1 and 2 of Sequence Listing show the nucleotide sequences for each of primer P5 and primer P6) were synthesized for transferrin receptor; primer P1 and primer P2 (SEQ ID NOs: 3 and 4 of Sequence Listing show the nucleotide sequences for each of primer P1 and primer P2) were synthesized for cytokeratin; primer P7 and primer P8 (SEQ ID NOs: 5 and 6 of Sequence Listing show the nucleotide sequences for each of primer P7 and primer P8) were synthesized for β-actin, respectively.

Twenty microliters of a reverse transcription reaction mixture comprising 50 ng of RNA, or 50 ng of DNA, and oligo dT adaptor primer attached to the kit was prepared by using RNA PCR Kit (AMV) Ver. 2.1 (manufactured by Takara Shuzo Co., Ltd.). The substrate nucleotides had seven kinds of compositions: One having the composition as described in the kit manual (Reaction Mixture 1); one in which dGTP was substituted by a mixture of dGTP and dITP (manufactured by Boehringer Mannheim) having each of the compositions shown in Table 1 (Reaction Mixtures 2 to 6, shown in Table 1); and one having the composition.of Reaction Mixture 1 added with 62.5 µM each of 7-Deaza-dATP and 7-Deaza-dGTP (both manufactured by Boehringer Mannheim) (Reaction Mixture 7). Each of these reaction mixtures was set on PCR Thermal Cycler PERSONAL (manufactured by Takara Shuzo Co., Ltd.), and reverse transcription reaction was carried out at 50°C, 20 minutes - 5°C, 5 minutes.

**Table 1**

| | dGTP | dITP |
|---|---|---|
| Reaction Mixture 2 | 1.75 mM | 0.25 mM |
| Reaction Mixture 3 | 1.5 mM | 0.5 mM |
| Reaction Mixture 4 | 1 mM | 1 mM |
| Reaction Mixture 5 | 0.5 mM | 1.5 mM |
| Reaction Mixture 6 | 0.25 mM | 1.75 mM |

The reaction mixture for PCR containing the primer pairs corresponding to each target mRNA was then prepared by using the above kit. Further, there were also prepared reaction mixtures by adding formamide to each of the reaction mixtures for PCR (the concentrations of formamide in each of the reaction mixtures for PCR being 6.25% by weight for transferrin receptor, 7.5% by weight for cytokeratin, and 13.75% by weight for β-actin). To the solution obtained after the reverse transcription reaction mentioned above was added 80 µl of these reaction mixtures, to make up a total volume of 100 µl of the reaction mixture. The resulting mixture was set on PCR Thermal Cycler PERSONAL, and 30 cycles of PCR were carried out, one cycle consisting of 82°C, 1 minute - 45°C, 1 minute - 72°C, 1 minute.

Eight microliters of the reaction mixture obtained as above was subjected to 3% agarose gel electrophoresis using NuSieve 3:1 Agarose (manufactured by FMC). The amount of the product in each reaction mixture after termination of reaction was evaluated by subjecting the agarose gel after electrophoresis to ethidium bromide staining and comparing the fluorescence intensity emitted by ultraviolet ray irradiation. Of the obtained results, those in which RNA was added as a template in the reverse transcription reaction mixture are shown in Table 2.

**Table 2**

| Target | Reverse Transcription Reaction Mixture | Formamide - | Formamide + |
|---|---|---|---|
| Transferrin | Reaction | - | ++ |
| | Mixture 1 | | |
| Receptor | Reaction | - | + |
| | Mixture 2 | | |
| | Reaction | - | ++ |
| | Mixture 3 | | |
| | Reaction | + | ++ |
| | Mixture 4 | | |
| | Reaction | + | + |
| | Mixture 5 | | |
| | Reaction | - | ± |
| | Mixture 6 | | |
| | Reaction | - | ++ |
| | Mixture 7 | | |
| Cytokeratin | Reaction | - | - |
| | Mixture 1 | | |
| | Reaction | - | - |
| | Mixture 2 | | |
| | Reaction | - | - |
| | Mixture 3 | | |
| | Reaction | - | + |
| | Mixture 4 | | |
| | Reaction | - | ± |
| | Mixture 5 | | |
| | Reaction | ± | ± |
| | Mixture 6 | | |
| | Reaction | - | + |
| | Mixture 7 | | |
| β-Actin | Reaction | - | ++ |
| | Mixture 1 | | |
| | Reaction | - | ± |
| | Mixture 2 | | |
| | Reaction | - | ++ |
| | Mixture 3 | | |
| | Reaction | - | ++ |
| | Mixture 4 | | |
| | Reaction | - | ± |
| | Mixture 5 | | |
| | Reaction | - | - |
| | Mixture 6 | | |
| | Reaction | - | ++ |
| | Mixture 7 | | |

| | | | |
|---|---|---|---|
| -: No amplification was found. | | | |
| ±: Slight amplification was found. | | | |
| +: Some amplification was found. | | | |
| ++: Strong amplification was found. | | | |

When PCR was carried out in a reaction system in which formamide was not added, only a slight amount of an amplified product was obtained in very limited reaction mixtures, whereas in a reaction system in which formamide was added, amplified products could be obtained for any of target RNAs by using Reaction Mixture 4 in the reverse transcription reaction. Further, in a reaction system in which formamide was added, amplified products could be obtained for all of the target RNAs by using Reaction Mixture 7 comprising two kinds of nucleotide analogs. In addition, when the target was cytokeratin, a desired amplified product could not be obtained when formamide was added during PCR without using a nucleotide analog. Incidentally, when DNA was added as a template to the reverse transcription reaction mixture, the amplified products could not be obtained, regardless of the compositions of the reaction mixtures.

It was shown from these results that various DNA fragments derived from RNAs can be efficiently amplified under given temperature conditions when PCR was carried out by adding formamide during PCR and using as a nucleotide analog a given amount of dITP or a given amount of a combination of 7-Deaza-dATP and 7-Deaza-dGTP. In addition, since amplification does not take place under these conditions by the use of DNA as a template, it was also elucidated that DNA fragments by the use of RNA as a template were selectively amplified.

### Example 3: Comparison of Combinations of Nucleotide Analogs

The following experiment was carried out with a region of about 2 kb in human transferrin receptor mRNA as a target.

Twenty microliters of a reverse transcription reaction mixture comprising 50 ng of RNA derived from human HL-60 cells, or 50 ng of DNA and the above primer P4 (SEQ ID NO: 7 of Sequence Listing shows the nucleotide sequence of primer P4), and the following nucleotide analogs, each at a concentration of 62.5 µM, was prepared by using RNA PCR Kit (AMV) Ver. 2.1.
- Reaction Mixture 1:: 7-Deaza-dGTP
- Reaction Mixture 2:: 7-Deaza-dATP
- Reaction Mixture 3:: dITP
- Reaction Mixture 4:: dITP, 7-Deaza-dATP
- Reaction Mixture 5:: 7-Deaza-dGTP, 7-Deaza-dATP

Each of these reaction mixtures was set on PCR Thermal Cycler PERSONAL, and the reverse transcription reaction was carried out under the conditions of 50°C, 20 minutes - 5°C, 5 minutes.

A reaction mixture.for PCR comprising primer P4 and primer P9 (SEQ ID NO: 8 of Sequence Listing shows the nucleotide sequence of primer P9) was prepared by using RNA PCR Kit mentioned above. Eighty microliters of the reaction mixture was added to a solution obtained after the reverse transcription reaction as mentioned above to make up a total volume of the reaction mixture of 100 µl. The resulting mixture was set on PCR Thermal Cycler PERSONAL, and 30 cycles of PCR were carried out, one cycle consisting of 84°C, 1 minute - 60°C, 30 seconds - 72°C, 2 minutes.

Eight microliters of the resulting reaction mixture was subjected to 1% agarose gel electrophoresis prepared with Agarose L03 (manufactured by Takara Shuzo Co., Ltd.), and evaluated in the same manner as in Example 2. As a result, in all reaction conditions, only amplified fragments by the use of RNAs as templates were found, and the highest amplification efficiency was obtained when Reaction Mixture 2 was used among reaction systems in which only one kind of the nucleotide analog was added. It was shown from the above results that the amplification efficiency differs depending upon the kinds of nucleotide analogs used. In addition, in Reaction Mixtures 4 and 5 using two kinds of nucleotide analogs, both reaction mixtures showed higher amplification efficiency, as compared to any of the reaction systems in which one kind of the nucleotide analog was added. In addition, in the reaction mixture to which DNA was added as a template, no amplified product was found regardless of the composition.

### Example 4: RT-PCR Using Various Enzymes for Reverse Transcription Reaction

Twenty microliters of a reverse transcription reaction mixture comprising 500 ng of RNA or 250 ng of DNA, each prepared from human HL-60 cells, and primer P4, and added with 7-Deaza-dATP and 7-Deaza-dGTP, each at a final concentration of 62.5 µM was prepared by using RNA PCR Kit (AMV) Ver. 2.1 (AMV-RTase), BcaBEST RNA PCR Kit [*Bacillus caldotenax*-derived DNA polymerase (BcaBEST), manufactured by Takara Shuzo Co., Ltd.], or MMLV-RTase (manufactured by LIFE TECHNOLOGIES) in accordance with the manual for each of the enzymes. Each of these reaction mixtures was set on PCR Thermal Cycler PERSONAL, and the reverse transcription reaction was carried out under the following program.

### Program for Reverse Transcription Reaction

- AMV RTase:: 50°C, 20 minutes - 5°C, 5 minutes
- BcaBEST:: treating at 65°C, 1 minute - 30°C, 1 minute; thereafter, heating to 65°C over a period of 15 minutes; subsequently 65°C, 15 minutes - 80°C, 2 minutes - 5°C, 5 minutes
- MMLV-RTase:: 42°C , 50 minutes - 70°C, 15 minutes - 5°C, 5 minutes

Subsequently, a reaction mixture for PCR was prepared by using the solution obtained after the reverse transcription reaction. The reaction mixture for PCR comprising primers P4 and P3 (SEQ ID NOs: 7 and 9 of Sequence Listing show the nucleotide sequences of each of primer P4 and primer P3), the reaction mixture being further added with formamide was prepared by using BcaBEST RNA PCR Kit when the reverse transcription reaction was carried out using BcaBEST, or using RNA PCR Kit (AMV) Ver. 2.1 when a reverse transcriptase other than BcaBEST was used, referring to the instruction manual for each kit. The concentration of formamide in the reaction mixture for PCR was 2.5% by weight when BcaBEST RNA PCR Kit was used, or 6.25% by weight when RNA PCR Kit (AMV) Ver. 2.1 was used. Eighty microliters of each of these reaction mixtures for PCR was added to the solution after the reverse transcription reaction mentioned above to make up a total volume of 100 µl of a reaction mixture. The resulting reaction mixture was set on PCR Thermal Cycler PERSONAL, and 30 cycles of PCR were carried out, one cycle consisting of 82°C, 30 seconds - 60°C , 30 seconds - 72°C, 1 minute.

After the termination of reaction, 8 µl of the reaction mixture was taken and subjected to 1% agarose gel electrophoresis prepared with Agarose L03 (manufactured by Takara Shuzo Co., Ltd.), and evaluated in the same manner as in Example 2. As a result, regardless of the enzymes used, no amplified fragments were found in the reaction mixture in which DNA was added as a template. By contrast, amplified fragments of the same size (964 bp) were found in all of the reaction mixtures in which RNA was added.

### Example 5: Application to RT-PCR by One Reaction Mixture

RT-PCR in which the reverse transcription reaction and PCR are carried out in one reaction mixture was carried out with a region consisting of 252 bases in human cytokeratin mRNA as a target.

By using GeneAmp EZ rTth RNA PCR Kit (manufactured by Perkin-Elmer), three kinds of reaction mixtures, specifically (1) one having the composition as described in the kit manual; (2) the composition added with dITP at a final concentration of 300 µM; and (3) the composition added with 7-Deaza-dATP and 7-Deaza-dGTP, at a final concentration of 150 µM each, and formamide at a final concentration of: 2% by weight were prepared. To each of the reaction mixtures were added primer P1 and primer P2 mentioned above; 1 µg of RNA derived from human HT-29 cells, and 0, 50, 100 or 200 ng of DNA derived from the cells. For each of the resulting reaction mixtures, RT-PCR was carried out under two patterns of the following conditions using PCR Thermal Cycler PERSONAL.

### Condition 1:

| | |
|---|---|
| Reverse transcription reaction (60°C, 30 minutes) | once |
| Denaturation (94°C, 2 minutes) | once |
| PCR (94°C, 30 seconds - 60°C, 30 seconds - 72°C, 1 minute) | 30 cycles |

### Condition 2:

| | |
|---|---|
| Reverse transcription reaction (60°C, 30 minutes) | once |
| Denaturation (82°C, 2 minutes) | once |
| PCR (82°C, 30 seconds - 60°C, 30 seconds - 72°C, 1 minute) | 30 cycles |

Eight microliters of each of the reaction mixtures for PCR as obtained above was subjected to 3% agarose gel electrophoresis using NuSieve 3:1 Agarose, and the formed amplified product was analyzed. As a result, under Condition 1, regardless of the composition of the reaction mixtures, DNA-derived amplified product (604 bp) appeared as well as RNA-derived amplified product (251 bp), and its amplification level was dependent on the concentration of DNA added. Under Condition 2, although not any of the amplified products could be obtained in the reaction mixture (1), only amplified products in which RNA was used as a template were obtained in the reaction mixtures (2) and (3). In this case, as compared to the reaction mixture (2), the reaction mixture (3) showed higher amplification efficiency.

### Example 6: RT-PCR Using Formamide and Dimethyl Sulfoxide in Combination

The effects to RT-PCR were compared between a case where only formamide was added to a reaction mixture, and a case where formamide and dimethyl sulfoxide were added to a reaction mixture.

Each of the target mRNAs and the primers used for amplification were as follows: A region consisting of 275 bases (GC content: 58%) in β-actin mRNA: primer P7 and primer P8; a region consisting of 325 bases (GC content: 61%) in tumor necrosis factor (TNF) mRNA: primer P10 and primer P11 (SEQ ID NOs: 10 and 11 of Sequence Listing show the nucleotide sequences for each of primer. P10 and primer P11); and a region consisting of 448 bases in Mcl mRNA (GC content: 51%), apoptosis-associated cytokine: primer P12 and primer P13 (SEQ ID NOs: 12 and 13 of Sequence Listing show the nucleotide sequences for each of primer P12 and primer P13).

By using One Step RNA PCR Kit (manufactured by Takara Shuzo Co., Ltd.), there was prepared a reaction mixture comprising 1 µg of RNA derived from human culture cells HL-60 and a primer corresponding to each target mentioned above and added with 100 µM each of 7-Deaza-dGTP and 7-Deaza-dATP according to the manual of the kit. Further, in addition to this reaction mixture, ones in which formamide was added to have a final concentration of 2, 4, 6, 8 or 10% by weight, respectively, to each reaction mixture, and one in which formamide was added to have a final concentration of 2% by weight and dimethyl sulfoxide was added to have a final concentration of 3% by weight to the reaction mixture, i.e. seven reaction mixtures in total, were prepared against each of the targets. Each of the resulting reaction mixtures was set on PCR Thermal Cycler PERSONAL, and RT-PCR was carried out under the following conditions.

| | |
|---|---|
| Reverse transcription reaction (50°C, 20 minutes) | once |
| Denaturation (85°C, 2 minutes) | once |
| PCR (85°C, 1 minute - 45°C, 1 minute - 72°C, 1 minute) | 30 cycles |

Eight microliters of each of the reaction mixtures obtained as above was subjected to 3% agarose gel elctrophoresis using NuSieve 3:1 Agarose, and the formed amplified product was analyzed in the same manner as in Example 2. The results thereof are shown in Table 3.

**Table 3**

| Target | | β-Actin | TNF | Mcl |
|---|---|---|---|---|
| No addition of organic solvent | | - | - | - |
| Formamide | 2% by weight | + | - | - |
| | 4% by weight | + | ± | + |
| | 6% by weight | + | + | - |
| | 8% by weight | ± | - | - |
| | 10% by weight | - | - | - |
| Formamide 2% by weight and Dimethyl Sulfoxide 3% by weight | | ++ | ++ | ++ |

| | | | | |
|---|---|---|---|---|
| -: No amplification was found. | | | | |
| ±: Slight amplification was found. | | | | |
| +: Some amplification was found. | | | | |
| ++: Strong amplification was found. | | | | |

As shown in Table 3, it was demonstrated that while the concentration of formamide needs to be adjusted for each target RNA in order to obtain excellent amplification efficiency when using only formamide, any of the targets are efficiently amplified with a single reaction mixture when formamide was used in combination with dimethyl sulfoxide.

### Example 7: Kit

As the kit usable for the method for amplifying DNA of the present invention, there was prepared the following kit for 20 reactions.

| | | |
|---|---|---|
| 10 × Buffer for reverse transcription reaction | | 40 µl |
| | 100 mM Tris-HCl (pH 8.3) | |
| | 500 mM KCl | |
| | 100 mM MgCl₂ | |
| | 100 mM DTT | |
| | 500 µM 7-Deaza-dGTP | |
| | 500 µM 7-Deaza-dATP | |
| | 10 mM dNTP | |
| | | |
| AMV Reverse Transcriptase (5 U/µl) | | 20 µl |
| | | |
| 2 × Buffer for PCR Reaction | | 800 µl |
| | 20 mM Tris-HCl (pH 8.3) | |
| | 100 mM KCl | |
| | 5% by weight Formamide | |
| | 5% by weight Dimethyl Sulfoxide | |
| | | |
| Taq DNA Polymerase (5 U/µl) | | 20 µl |

### INDUSTRIAL APPLICABILITY

According to the present invention, DNA having a nucleotide sequence derived from RNA can be selectively amplified. By using the method of the present invention, a DNA fragment derived from RNA can be amplified without previously purifying RNA in a sample, thereby imparting simplification of the experimental procedures and improvement in reproducibility.

## Claims

1. A method for amplifying a DNA by the use of a DNA fragment comprising a nucleotide analog as a template in the presence of nucleotide analogs, **characterized in that** the method for amplifying a DNA is carried out in the presence of (a) 7-Deaza-dGTP and 7-Deaza-dATP or (b)dITP and 7-Deaza-dATP;
and, optionally, in the presence of a compound for lowering *Tm* value of a double-stranded nucleic acid.

2. A method for amplifying a DNA by the use of a DNA fragment comprising a nucleotide analog as a template in the presence of nucleotide analogs, **characterized in that** the method for amplifying a DNA is carried out in the presence of one or more kinds of nucleotide analogs and a compound for lowering *Tm* value of a double-stranded nucleic acid.

3. The method for amplifying a DNA according to claim 1 or 2, **characterized in that** amplification of a DNA is carried out by polymerase chain reaction.

4. The method for amplifying a DNA according to any one of claims 1 to 3, **characterized in that** the DNA fragment is a cDNA prepared by reverse transcription reaction using an RNA as a template in the presence of nucleotide analogs.

5. The method for amplifying a DNA according.to claim 4, **characterized in that** the DNA fragment is a cDNA prepared by reverse transcription reaction using an RNA as a template in the presence of two or more nucleotide analogs.

6. The method for amplifying a DNA according to claims 2, **characterized by** the use, as the nucleotide analog, of a nucleotide analog having a property of lowering *Tm* value of a double-stranded nucleic acid to which the nucleotide analog is to be incorporated.

7. The method for amplifying a DNA according to claim 2, **characterized by** the use of a nucleotide analog to be incorporated in a DNA strand in place of dATP or dTTP, and the use of a nucleotide analog to be incorporated in a DNA strand in place of dCTP or dGTP.

8. The method for amplifying a DNA according to claims 2, wherein the nucleotide analog is selected from the group consisting of 7-Deaza-dGTP, 7-Deaza-dATP, dITP and hydroxymethyl dUTP.

9. The method for amplifying a DNA according to any one of claims 1 to 8, **characterized in that** one or more kinds of compounds selected from the group consisting of formamide, dimethyl sulfoxide and trimethyl glycine are used as the compound for lowering Tm value of a double-stranded nucleic acid.

10. A kit for amplifying a DNA in the presence of a nucleotide analog by the use of a DNA fragment comprising a nucleotide analog as a template, **characterized in that** the kit comprises (a) 7-Deaza-dGTP and 7-Deaza-dATP or (b) dITP and 7-Deaza-dATP.

11. A kit for amplifying a DNA in the presence of a nucleotide analog by the use of a DNA fragment comprising a nucleotide analog as a template, **characterized in that** the kit comprises one or more kinds of nucleotide analogs and a compound for lowering *Tm* value of a double-stranded nucleic acid.

12. The kit according to claim 10 or 11, **characterized in that** the kit comprises a reagent for synthesizing a cDNA which is complementary to an RNA in the presence of nucleotide analogs.

13. The kit according to claims 11, **characterized in that** the kit comprises as the nucleotide analog a nucleotide analog having a property of lowering Tm value of a double-stranded nucleic acid to which the nucleotide analog is to be incorporated.

14. The kit according claim 11, **characterized in that** the kit comprises a nucleotide analog to be incorporated in a DNA strand in place of dATP or dTTP, and a nucleotide analog to be incorporated in a DNA strand in place of dCTP or dGTP.

15. The kit according to any one of claims 11 to 14, wherein the nucleotide analog is selected from the group consisting of 7-Deaza-dGTP, 7-Deaza-dATP, dITP and hydroxymethyl dUTP.

16. The kit according to any one of claims 10 to 15, **characterized in that** the kit comprises, one or more compounds for lowering *Tm* value of a double-stranded nucleic acid selected from the group consisting of formamide, dimethyl sulfoxide and trimethyl glycine.

## Patentansprüche

1. Verfahren zur Amplifikation einer DNA durch Verwendung eines DNA-Fragments, umfassend ein Nukleotidanalog als Matrize in Gegenwart von Nukleotidanalogen, **dadurch gekennzeichnet, dass** das Verfahren zur Amplifikation einer DNA in Gegenwart von (a) 7-Deaza-dGTP und 7-Deaza-dATP oder (b) dITP und 7-Deaza-dATP durchgeführt wird; und wahlweise in Gegenwart einer Verbindung zum Senken des Tm-Werts einer doppelsträngigen Nukleinsäure.

2. Verfahren zur Amplifikation einer DNA durch Verwendung eines DNA-Fragments, umfassend ein Nukleotidanalog als Matrize in Gegenwart der Nukleotidanalogen, **dadurch gekennzeichnet, dass** das Verfahren zur Amplifikation einer DNA in Gegenwart von ein oder mehr Nukleotidanalog-Arten und einer Verbindung zum Senken des Tm-Werts einer doppelsträngigen Nukleinsäure durchgeführt wird.

3. Verfahren zur Amplifikation einer DNA nach Anspruch 1 oder 2, **dadurch gekennzeichnet, dass** die Amplifikation einer DNA mittels Polymerasekettenreaktion durchgeführt wird.

4. Verfahren zur Amplifikation einer DNA nach einem der Ansprüche 1 bis 3, **dadurch gekennzeichnet, dass** das DNA-Fragment eine cDNA ist, die durch Umkehrtranskriptionsreaktion unter Verwendung einer RNA als Matrize in Gegenwart von Nukleotidanalogen hergestellt wird.

5. Verfahren zur Amplifikation einer DNA nach Anspruch 4, **dadurch gekennzeichnet, dass** das DNA-Fragment eine cDNA ist, die durch Umkehrtranskriptionsreaktion unter Verwendung einer RNA als Matrize in Gegenwart von zwei oder mehr Nukleotidanalogen durchgeführt wird.

6. Verfahren zur Amplifikation einer DNA nach Anspruch 2, **gekennzeichnet durch** die Verwendung eines Nukleotidanalogs mit der Eigenschaft, den Tm-Wert einer doppelsträngigen, das Nukleotidanalog aufzunehmenden Nukleotidsäure zu senken, als Nukleotidanalog.

7. Verfahren zur Amplifikation einer DNA nach Anspruch 2, **gekennzeichnet durch** die Verwendung eines anstelle von dATP oder dTTP in einen DNA-Strang aufzunehmenden Nukleotidanalogs und die Verwendung eines anstelle von dCTP oder dGTP in einen DNA-Strang aufzunehmenden Nukleotidanalogs.

8. Verfahren zur Amplifikation einer DNA nach Anspruch 2, wobei das Nukleotidanalog aus der Gruppe bestehend aus 7-Deaza-dGTP, 7-Deaza-dATP, dITP und Hydroxymethyl-dUTP ausgewählt wird.

9. Verfahren zur Amplifikation einer DNA nach einem der Ansprüche 1 bis 8, **dadurch gekennzeichnet, dass** ein oder mehr Arten von Verbindungen, die aus der Gruppe bestehend aus Formamid, Dimethylsulfoxid und Trimethylglycin ausgewählt sind, als Verbindung zum Senken des Tm-Werts einer doppelsträngigen Nukleinsäure verwendet werden.

10. Kit zur Amplifikation einer DNA in Gegenwart eines Nukleotidanalogs durch Verwendung eines DNA-Fragments, umfassend ein Nukleotidanalog als Matrize, **dadurch gekennzeichnet, dass** der Kit (a) 7-Deaza-dGTP und 7-Deaza-dATP oder (b) dITP und 7-Deaza-dATP umfasst.

11. Kit zur Amplifikation einer DNA in Gegenwart eines Nukleotidanalogs durch Verwendung eines DNA-Fragments, umfassend ein Nukleotidanalog als Matrize, **dadurch gekennzeichnet**, das der Kit ein oder mehr Arten von Nukleotidanalogen und eine Verbindung zum Senken des Tm-Werts einer doppelsträngigen Nukleinsäure umfasst.

12. Kit nach Anspruch 10 oder 11, **dadurch gekennzeichnet, dass** der Kit ein Reagens zum Synthetisieren einer cDNA, die komplementär zu einer RNA ist, in Gegenwart von Nukleotidanalogen umfasst.

13. Kit nach Anspruch 11, **dadurch gekennzeichnet, dass** der Kit als Nukleotidanalog ein Nukleotidanalog umfasst, das die Eigenschaft hat, den Tm-Wert einer doppelsträngigen Nukleinsäure, in die das Nukleotidanalog aufgenommen werden soll, zu senken.

14. Kit nach Anspruch 11, **dadurch gekennzeichnet, dass** der Kit ein Nukleotidanalog, das anstelle von dATP oder dTTP in einen DNA-Strang aufgenommen werden soll, und ein Nukleotidanalog umfasst, das anstelle von dCTP oder dGTP in einen DNA-Strang aufgenommen werden soll.

15. Kit nach einem der Ansprüche 11 bis 14, wobei das Nukleotidanalog aus der Gruppe bestehend aus 7-Deaza-dGTP, 7-Deaza-dATP, dITP und Hydroxymethyl-dUTP ausgewählt ist.

16. Kit nach einem der Ansprüche 10 bis 15, **dadurch gekennzeichnet, dass** der Kit ein oder mehr Verbindungen zum Senken des Tm-Werts einer doppelsträngigen Nukleinsäure umfasst, die aus der Gruppe bestehend aus Formamid, Dimethylsulfoxid und Trimethylglycin ausgewählt ist.

## Revendications

1. Méthode d'amplification d'un ADN par l'utilisation d'un fragment d'ADN comprenant un analogue nucléotidique en tant que matrice en présence d'analogues nucléotidiques, **caractérisée en ce que** la méthode d'amplification d'un ADN est effectuée en présence de (a) 7-Déaza-dGTP et 7-Déaza-dATP ou (b) dITP et 7-Déaza-dATP ;
et, le cas échéant, en présence d'un composé servant à abaisser la valeur de Tm d'un acide nucléique bicaténaire.

2. Méthode d'amplification d'un ADN par l'utilisation d'un fragment d'ADN comprenant un analogue nucléotidique en tant que matrice en présence d'analogues nucléotidiques, **caractérisée en ce que** la méthode d'amplification d'un ADN est mise en oeuvre en présence d'une ou plusieurs sortes d'analogues nucléotidiques et d'un composé servant à abaisser la valeur de Tm d'un acide nucléique bicaténaire.

3. Méthode d'amplification d'un ADN selon la revendication 1 ou 2, **caractérisée en ce que** l'amplification d'un ADN est mise en oeuvre par réaction en chaîne par polymérase.

4. Méthode d'amplification d'un ADN selon l'une quelconque des revendications 1 à 3, **caractérisée en ce que** le fragment d'ADN est un ADNc préparé par réaction de transcription inverse en utilisant un ARN en tant que matrice en présence d'analogues nucléotidiques.

5. Méthode d'amplification d'un ADN selon la revendication 4, **caractérisée en ce que** le fragment d'ADN est un ADNc préparé par une réaction de transcription inverse en utilisant un ARN en tant que matrice en présence de deux ou plusieurs analogues nucléotidiques.

6. Méthode d'amplification d'un ADN selon la revendication 2, **caractérisée par** l'utilisation en tant qu'analogue nucléotidique, d'un analogue nucléotidique ayant la propriété d'abaisser la valeur du Tm d'un acide nucléique bicaténaire auquel l'analogue nucléotidique doit être incorporé.

7. Méthode d'amplification d'un ADN selon la revendication 2, **caractérisée par** l'utilisation d'un analogue nucléotidique à incorporer dans un brin d'ADN à la place du dATP ou du dTTP, et l'utilisation d'un analogue nucléotidique à incorporer dans un brin d'ADN à la place du dCTP ou du dGTP.

8. Méthode d'amplification d'un ADN selon la revendication 2, dans laquelle l'analogue nucléotidique est choisi dans le groupe constitué du 7-Déaza-dGTP, du 7-Déaza-dATP, du dITP et de l'hydroxyméthyl-dUTP.

9. Méthode d'amplification d'un ADN selon l'une quelconque des revendications 1 à 8, **caractérisée en ce que** l'on utilise une ou plusieurs sortes de composés choisis dans le groupe constitué du formamide, du diméthyl sulfoxyde et de la triméthyl glycine en tant que composé servant à abaisser la valeur du Tm d'un acide nucléique bicaténaire.

10. Kit pour l'amplification d'un ADN en présence d'un analogue nucléotidique par l'utilisation d'un fragment d'ADN comprenant un analogue nucléotidique en tant que matrice, **caractérisé en ce que** le kit comprend (a) du 7-Déaza-dGTP et du 7-Déaza-dATP ou (b) du dITP et du 7-Déaza-dATP.

11. Kit pour l'amplification d'un ADN en présence d'un analogue nucléotidique par l'utilisation d'un fragment d'ADN comprenant un analogue nucléotidique en tant que matrice, **caractérisé en ce que** le kit comprend une ou plusieurs sortes d'analogues nucléotidiques et un composé pour abaisser la valeur du Tm d'un acide nucléique bicaténaire.

12. Kit selon la revendication 10 ou 11, **caractérisé en ce que** le kit comprend un réactif pour synthétiser un ADNc qui est complémentaire d'un ARN en la présence d'analogues nucléotidiques.

13. Kit selon la revendication 11, **caractérisé en ce que** le kit comprend en tant qu'analogue nucléotidique, un analogue nucléotidique ayant la propriété d'abaisser la valeur du Tm d'un acide nucléique bicaténaire auquel l'analogue nucléotidique doit être incorporé.

14. Kit selon la revendication 11, **caractérisé en ce que** le kit comprend un analogue nucléotidique à incorporer dans un brin d'ADN à la place du dATP ou du dTTP, et un analogue nucléotidique à incorporer dans un brin d'ADN à la place du dCTP ou du dGTP.

15. Kit selon l'une quelconque des revendications 11 à 14, dans lequel l'analogue nucléotidique est choisi dans le groupe constitué du 7-Déaza-dGTP, du 7-Déaza-dATP, du dITP et de l'hydroxyméthyl dUTP.

16. Kit selon l'une quelconque des revendications 10 à 15, **caractérisé en ce que** le kit comprend un ou plusieurs composés servant à abaisser la valeur du Tm d'un acide nucléique bicaténaire choisi dans le groupe constitué du formamide, du diméthyl sulfoxyde et de la triméthyl glycine.


### SEQUENCE LISTING

<110>Takara Shuzo Co., Ltd.
<120>A DNA amplification method and a kit therefor
<130>98-031-PCT
<140>PCT/JP98/03556
   <141>1998-08-10
<150>JP 9/231885
   <151>1997-08-14
<150>JP 9/305016
   <151>1997-10-21
<160>13
<210>1
   <211>20
   <212>DNA
   <213>Artificial Sequence
<220>
   <223>Description of the Artifical Sequence: Primer
<400>1
   ccacagtgtc tgtatcggag 20
<210>2
   <211>20
   <212>DNA
   <213>Artificial Sequence
<220>
   <223>Description of the Artifical Sequence: Primer
<400>2
   gtttccaact gccctatgac 20
<210>3
   <211>22
   <212>DNA
   <213>Artificial Sequence
<220>
   <223>Description of the Artifical Sequence: Primer
<400>3
   cgtctaacag tggaagctga tc 22
<210>4
   <211>22
   <212>DNA
   <213>Artificial Sequence
<220>
   <223>Description of the Artifical Sequence: Primer
<400>4
   catgacttca tacttctgcc tc 22
   <210>5
   <211>21
   <212>DNA
   <213>Artificial Sequence
<220>
   <223>Description of the Artifical Sequence: Primer
<400>5
   caagagatgg ccacggctgc t 21
   <210>6
   <211>21
   <212>DNA
   <213>Artificial Sequence
<220>
   <223>Description of the Artifical Sequence: Primer
<400>6
   tccttctgca tcctgtcggc a 21
   <210>7
   <211>20
   <212>DNA
   <213>Artificial Sequence
<220>
   <223>Description of the Artifical Sequence: Primer
<400>7
   gagactgtga gtagtgacac 20
   <210>8
   <211>20
   <212>DNA
   <213>Artificial Sequence
<220>
   <223>Description of the Artifical Sequence: Primer
<400>8
   ccatcccatc atcttggtac 20
<210>9
   <211>20
   <212>DNA
   <213>Artificial Sequence
<220>
   <223>Description of the Artifical Sequence: Primer
<400>9
   ctaagaagcg agcactgacc 20
<210>10
   <211>20
   <212>DNA
   <213>Artificial Sequence
<220>
   <223>Description of the Artifical Sequence: Primer
<400>10
   cagagggaag agttccccag 20
<210>11
   <211>20
   <212>DNA
   <213>Artificial Sequence
<220>
   <223>Description of the Artifical Sequence: Primer
<400>11
   ccttggtctg gtaggagacg 20
<210>12
   <211>21
   <212>DNA
   <213>Artificial Sequence
<220>
   <223>Description of the Artifical Sequence: Primer
<400>12
   cggcagtcgc tggagattat c 21
<210>13
   <211>21
   <212>DNA
   <213>Artificial Sequence
<220>
   <223>Description of the Artifical Sequence: Primer
<400>13
   tctaggtcct ctacatggaa g 21